# EUROPÄISCHE PATENTSCHRIFT

(11) **EP 2 261 717 B1**
(45) Veröffentlichungstag und Bekanntmachung des Hinweises auf die Patenterteilung: **05.08.2020**
(21) Anmeldenummer: 10075245.0
(22) Anmeldetag: 08.06.2010
(51) Int. Cl.: G02B 21/00, G02B 21/08, A61B 90/30, A61B 1/06, G02B 21/02, G02B 21/22

(54) **Lichtquellenanordnung für eine Beleuchtungsvorrichtung eines medizinisch-optischen Beobachtungsgeräts**
Light source assembly for an illumination device of a medical optical observation device
Agencement de sources lumineuses pour un dispositif d'éclairage d'un appareil d'observation optique médical

(30) Priorität: 09.06.2009 DE 102009024942
(43) Veröffentlichungstag der Anmeldung: 15.12.2010
(73) Patentinhaber: Carl Zeiss Meditec AG, 07745 Jena (DE)
(72) Erfinder: Lücke, Christian, 73447 Oberkochen (DE); Bausewein, Markus, 73431 Aalen (DE); Reimer, Peter, 73479 Ellwangen (DE)
(74) Vertreter: Theobald, Andreas

(56) Entgegenhaltungen:
- EP-A1- 1 510 847
- EP-A1- 1 691 229
- EP-A2- 2 030 560
- DE-A1- 19 739 428
- DE-A1-102006 047 724
- GB-A- 191 316 004
- US-A1- 2003 112 505

## Beschreibung

Die vorliegende Erfindung betrifft eine Lichtquellenanordnung für eine Beleuchtungsvorrichtung eines medizinisch-optischen Beobachtungsgeräts, welche eine Beleuchtungslichtquelle und eine Beleuchtungsoptik zur Beleuchtung eines Beobachtungsobjekts mit Beleuchtungslicht der Beleuchtungslichtquelle aufweist. Daneben betrifft die Erfindung eine Beleuchtungsvorrichtung für ein medizinisch-optisches Beobachtungsgerät und solch ein medizinisch-optisches Beobachtungsgerät.

Eine Beleuchtungsvorrichtung für ein Operationsmikroskop, welches als ophthalmologisches Operationsmikroskop ausgebildet ist, ist beispielsweise in DE 10 2007 041 003 A1 beschrieben. Darin werden die Beleuchtungssysteme im Operationsmikroskop über gespleißte Lichtleiter aus einer Halogen- oder Xenonlichtquelle bespeist. Damit sind die Beleuchtungsarten Koaxialbeleuchtung und Umfeldbeleuchtung jedoch nicht unabhängig voneinander regelbar. Im Falle der Verwendung mehrerer Lichtleiter ist zwar die getrennte Regelung grundsätzlich möglich, jedoch erhöht sich dadurch die Komplexität des Beleuchtungssystems.

Aus DE 20 2004 019 849 U1 und EP 0 661 020 A1 sind außerdem Beleuchtungsvorrichtungen bekannt, die für die Rotreflexbeleuchtung und die Umfeldbeleuchtung getrennte Lichtquellen vorsehen. In DE 20 2004 019 849 U1 ist außerdem erwähnt, dass Leuchtdioden als Lichtquelle Verwendung finden können. Über die praktische Ausgestaltung der Beleuchtungsvorrichtung bei Verwendung von Leuchtdioden als Lichtquellen ist jedoch nichts weiter ausgeführt.

DE 10 2006 047 724 A1 beschreibt ein Operationsmikroskop mit einer Beleuchtungsvorrichtung, welche eine Beleuchtungsoptik umfasst. Die Beleuchtungsoptik umfasst eine Beleuchtungslichtquelle in Form einer leistungsstarken Xenonlampe, deren Licht mittels eines Lichtleiters der Beleuchtungsoptik zugeführt wird.

US 2003/0112505 A1 beschreibt eine Beleuchtungsvorrichtung, die in einem Fluoreszenzmikroskop, einem industriellen Mikroskop, etc. Verwendung finden kann. Die Beleuchtungsoptik umfasst einen Linsentubus, in der ein Bild einer Lichtquelle erzeugt wird.

EP 1 510 847 A1 beschreibt ein Stereooperationsmikroskop mit Auflicht-Beleuchtungseinrichtung. Die Beleuchtungseinrichtung weist dabei eine Beleuchtungsoptik auf, der entweder das Licht der Lichtquelle selbst oder das Licht aus dem Austrittsende eines Lichtleiters zugeführt wird.

EP 2 030 560 A2 beschreibt eine sekundäre Lichtquelle mit einem Lichtleiter und einer primären Lichtquelle, deren Licht in das Eingangsende des Lichtleiters eingekoppelt wird.

Aufgabe der vorliegenden Erfindung ist es, eine vorteilhafte Lichtquellenanordnung für eine Beleuchtungsvorrichtung eines medizinisch-optischen Beobachtungsgerätes zur Verfügung zu stellen. Es ist eine weitere Aufgabe der vorliegenden Erfindung, eine vorteilhafte Beleuchtungsvorrichtung für ein medizinisch-optisches Beobachtungsgerät zur Verfügung zu stellen. Schließlich ist es eine Aufgabe der vorliegenden Erfindung, eine vorteilhafte Verwendung für eine Lichtquellenanordnung zur Verfügung zu stellen.

Die erste Aufgabe wird durch eine Lichtquellenanordnung nach Anspruch 1 gelöst, die zweite Aufgabe durch eine Beleuchtungsvorrichtung nach Anspruch 10 und die dritte Aufgabe durch die Verwendung einer Lichtquellenanordnung gemäß Anspruch 13.

Gemäß der Erfindung wird eine Lichtquellenanordnung für eine Beleuchtungsvorrichtung eines medizinisch-optischen Beobachtungsgerätes zur Verfügung gestellt, wobei die Beleuchtungsvorrichtung eine Beleuchtungslichtquelle und eine Beleuchtungsoptik zur Beleuchtung eines Beobachtungsobjekts mit Beleuchtungslicht der Beleuchtungslichtquelle aufweist. Die Lichtquellenanordnung umfasst wenigstens Lumineszenzstrahler, der eine Leuchtdiode ist, als primäre Lichtquelle für die Beleuchtungsvorrichtung. Die Lichtquellenanordnung umfasst weiterhin eine Abbildungsoptik, die ein Bild, insbesondere ein reelles Bild, des wenigstens einen Lumineszenzstrahlers mit einem definierten Vergrößerungsmaßstab erzeugt. Das Bild stellt die Beleuchtungslichtquelle für die Beleuchtungsvorrichtung dar. Als Abbildungsoptik kann im einfachsten Fall eine Sammellinse zur Anwendung kommen. Es sind aber vorzugsweise auch komplexere Optiken einsetzbar.

Die vorliegende Erfindung basiert auf der Erkenntnis, dass in Beleuchtungsvorrichtungen für medizinisch-optische Beobachtungsgeräte die bisher zum Einsatz kommenden Lichtquellen wie Austrittsenden von Lichtleitfasern, Glühlampen oder Gasentladungslampen nicht durch eine Leichtdiode ersetzt werden können, ohne entweder einen Qualitätsverlust der Beleuchtung herbeizuführen oder die Beleuchtungsoptik der Beleuchtungsvorrichtung an die neue Lichtquelle anzupassen. Beispielsweise bei einer Beleuchtungsvorrichtung mit dem Austrittsende einer Lichtleitfaser als Beleuchtungslichtquelle würde das Ersetzen des Austrittsendes durch eine Leuchtdiode aufgrund der unterschiedlichen Abstrahlcharakteristiken zur einer deutlichen Verringerung der über die Beleuchtungsoptik übertragene Beleuchtungslichtmenge führen, wenn die Beleuchtungsoptik nicht an die neue Lichtquelle angepasst wird. Da eine Leuchtdiode jedoch einen deutlich größeren Abstrahlwinkel als das Austrittsende einer Lichtleitfaser aufweist, müsste die Beleuchtungsoptik mit einer größeren numerischen Eintrittsapertur ausgestaltet werden, was die optischen Komponenten in ihrem Durchmesser vergrößern würde. Eine derartige Vergrößerung bedeutet jedoch mehr Bauraum, was die Beleuchtungsvorrichtung sperriger machen würde. Zudem ist das Ersetzen der optischen Komponenten in bestehenden Beleuchtungsvorrichtungen nicht ohne weiteres möglich. Erfindungsgemäß wird daher nicht die Leuchtdiode selbst als Beleuchtungslichtquelle verwendet, sondern ein Bild der Beleuchtungslichtquelle, das mittels einer Abbildungsoptik mit einem definierten Vergrößerungsmaßstab im Bereich zwischen 1,5x und 2,5x erzeugt wird. Damit lässt sich das Bild des wenigstens einen Lumineszenzstrahlers optimal an die Beleuchtungsoptik der Beleuchtungsvorrichtung anpassen.

Im zuvor erwähnten Beispiel mit dem Austrittsende einer Lichtleitfaser und der LED liegen die folgenden Verhältnisse vor: Der Abstrahlwinkel einer Lichtleitfaser beträgt ca. ± 34°. Der Abstrahlwinkel einer Leuchtdiode, die ein als Lichtquelle besonders geeigneter Lumineszenzstrahler ist, beträgt hingegen ± 60°, also knapp das Doppelte der Lichtleitfaser. Die Beleuchtungsoptik der Beleuchtungsvorrichtung ist jedoch an das Übertragen von Beleuchtungslicht mit einem Öffnungswinkel, wie ihn die Lichtleitfaser bietet, angepasst. In der erfindungsgemäßen Lichtquellenanordnung ist die Abbildungsoptik so ausgebildet, dass der Abbildungsmaßstab des Bildes der Leuchtdiode zwischen 1,5x und 2,5x liegt, also etwa 2x beträgt. Dadurch ist die Leuchtfläche des Bildes gegenüber der ursprünglichen Leuchtdiode um den Faktor 2 vergrößert und gleichzeitig der Abstrahlwinkel um den Faktor 2 reduziert. Damit beträgt der Abstrahlwinkel ca. ± 30°, was in etwa dem Abstrahlwinkel einer Lichtleitfaser entspricht. Die Leuchtfläche der Leuchtdiode kann dabei so gewählt werden, dass das mit dem Faktor 2 vergrößerte Bild der Leuchtfläche etwa der

Leuchtfläche des Austrittsendes einer Lichtleitfaser entspricht. Wenn nun die erfindungsgemäße Lichtquellenanordnung so in Bezug auf die Beleuchtungsvorrichtung angeordnet wird, dass sich das Bild der um den Faktor 2 vergrößerten Leuchtdiode dort befindet, wo sich sonst das Austrittsende der Lichtfaser befinden würde, kann das Licht der Leuchtdiode, das heißt das Licht ihres Bildes, genau so gut in die an die Lichtleitfaser angepasste Beleuchtungsoptik eingekoppelt werden, wie das Licht der Lichtleitfaser selbst. Die erfindungsgemäße Lichtquellenanordnung lässt sich daher mit bestehenden Beleuchtungsvorrichtungen verwenden, ohne dass bei diesen eine Anpassung an die neue Lichtquelle vorgenommen werden müsste.

Als Abbildungsoptik kann vorteilhafterweise ein Doppelkollektor zur Anwendung kommen. Ein solcher bietet die Möglichkeit, dass zwischen den beiden Linsen des Doppelkollektors ein paralleler Strahlengang vorliegt. Auf diese Weise kann das Bild des wenigstens einen Lumineszenzstrahlers in einem beliebigen Abstand vom Lumininzenzstrahler erzeugt werden. Unter Verwendung Licht lenkender Elemente wie etwa Spiegel oder Prismen ergibt sich dann eine große Freiheit beim Positionieren des wenigstens einen Lumineszenzstrahlers. Dieser kann daher auch in einer größeren Entfernung von der Beleuchtungsvorrichtung angeordnet sein. Außerdem stehen dann insgesamt wenigstens vier Linsenflächen zur Verfügung, die zum Erzeugen einer optimierten Abbildungsqualität geeignet gewählt werden können.

In einer Weiterbildung der Erfindung weist die Abbildungsoptik der Lichtquellenanordung wenigsten eine asphärische Linse auf, was im Hinblick auf die Korrektur von Abbildungsfehlern vorteilhaft sein kann, insbesondere im Hinblick auf die Korrektur einer sphärischen Aberration.

Zudem kann die Abbildungsoptik eine achromatische oder apochromatische Linse umfassen, um Farbfehler in der Abbildung des Lumineszenzstrahlers gering zu halten. Dies ist insbesondere vorteilhaft, wenn ein breitbandiges Licht emittierender Luminizenzstrahler wie etwa eine weiße Leuchtdiode zur Anwendung kommt. Statt eines breitbandigen Lichtes emittierenden Lumineszenzstrahlers kann aber auch ein schmalbandiges Licht emittierender Lumineszenzstrahler zur Anwendung kommen, beispielsweise wenn eine Beleuchtung in einem schmalen spektralen Bereich erfolgen soll. Ein schmalbandiges Licht emittierender Lumineszenzstrahler kann aber auch zur Anwendung kommen, wenn die Beleuchtung eines Beobachtungsobjekts mit breitbandigem Licht erfolgen soll. In diesem Fall umfasst die Lichtquellenanordnung ein Konverterelement, also ein selbständiges Teil, das nicht in den Luminizenzstrahler integriert ist, welches mit einem Konverterleuchtstoff zum Konvertieren wenigstens eines Teils des schmalbandigen Lichtes des Lumineszenzstrahlers versehen ist. Das Konverterelement ist dann zwischen dem wenigstens einen Lumineszenzstrahler und dem Bild des wenigsten einen Luminizenzstrahlers in die Lichtquellenanordnung eingebracht oder einbringbar, beispielsweise zwischen der Abbildungsoptik und dem Bild des wenigstens einen Lumineszenzstrahlers. In einer derartigen Ausgestaltung der Lichtquellenanordnung kann durch Austausch des Konverterelementes gegen ein Konverterelement mit einem anderen Konverterleuchtstoff die spektrale Charakteristik des Bildes der Lichtquelle verändert werden, beispielsweise um Licht mit einer bestimmten Farbtemperatur zur Verfügung zu stellen.

Das Konverterelement kann eine dem Lumineszenzstrahler zugewandte Eintrittsfläche für das vom Lumineszenzstrahler ausgehende Licht aufweisen, die mit einer dichroitischen Schicht versehen ist, welche für in das Konverterelement eintretendes Licht mit der Wellenlängenverteilung des von dem Lumineszenzstrahler emittierten Lichtes transparent ist. Für in Richtung auf den Lumineszenzstrahler gerichtetes konvertiertes Licht ist diese dichroitische Schicht hingegen hoch reflektiv. Auf diese Weise kann verhindert werden, dass konvertiertes Licht in Richtung auf den Lumineszenzstrahler aus dem Konverterelement austritt und so für die Beleuchtung verloren ist.

Eine erfindungsgemäße Beleuchtungsvorrichtung für ein medizinisch-optisches Beobachtungsgerät ist mit wenigstens einer erfindungsgemäßen Lichtquellenanordung ausgestattet. Die Verwendung einer erfindungsgemäßen Lichtquellenanordnung mit einer LED als Lumineszenzstrahler als primärer Lichtquelle bietet gegenüber der Verwendung von Halogen- und Xenon-Lampen einen erheblichen Preisvorteil und außerdem eine längere Haltbarkeit. Im Vergleich zur Verwendung einer Lichtleitfaser als Lichtquelle bieten Leuchtdioden den Vorteil einer homogeneren Lichtausbreitung.

Wenn die Beleuchtungsvorrichtung wenigstens zwei Beleuchtungslichtquelien aufweist, können diese insbesondere von derselben Lichtquellenanordnung gebildet sein. Mit anderen Worten, die Lichtquellenanordnung umfasst dann wenigstens zwei Lumineszenzstrahler, die mit derselben Abbildungsoptik abgebildet werden. Alternativ besteht aber auch die Möglichkeit, zwei getrennte Lichtquellenanordnungen als Beleuchtungslichtquellen zu verwenden. Mit anderen Worten, es können wenigstens zwei Lichtquellenanordnungen mit jeweils einem eigenen Luminizenzstrahler und einer eigenen Abbildungsoptik zu Anwendung kommen.

Ein erfindungsgemäßes medizinisch-optisches Beobachtungsgerät, das beispielsweise als Endoskop oder insbesondere als Operationsmikroskop ausgebildet sein kann, ist mit einer erfindungsgemäßen Beleuchtungsvorrichtung ausgestattet. Die hierbei erzielbaren Vorteile ergeben sich aus die bereits mit Bezug auf die erfindungsgemäße Beleuchtungsvorrichtung und die mit Bezug auf die erfindungsgemäße Lichtquellenanordnung beschriebenen Vorteilen.

Weiter Merkmale, Eigenschaften und Vorteile der vorliegenden Erfidnung ergeben sich aus der nachfolgenden Beschreibung von Ausführungsbeispielen unter Bezugnahme auf die beiliegenden Figuren.
- Figur 1: zeigt ein erstes Ausführungsbeispiel für eine erfindungsgemäße Lichtquellenvorrichtung.
- Figur 2: zeigt ein zweites Ausführungsbeispiel für eine erfindungsgemäße Lichtquellenvorrichtung.
- Figur 3: zeigt ein drittes Ausführungsbeispiel für eine erfindungsgemäße Lichtquellenvorrichtung.
- Figur 4: zeigt ein erstes Ausführungsbeispiel für ein medizinischoptisches Beobachtungsgerät mit einer erfindungsgemäßen Beleuchtungsvorrichtung.
- Figur 5: zeigt ein zweites Ausführungsbeispiel für ein medizinischoptisches Beobachtungsgerät mit einer erfindungsgemäßen Beleuchtungsvorrichtung.
- Figur 6: zeigt ein drittes Ausführungsbeispiei für ein medizinischoptisches Beobachtungsgerät mit einer erfindungsgemäßen Beleuchtungsvorrichtung.

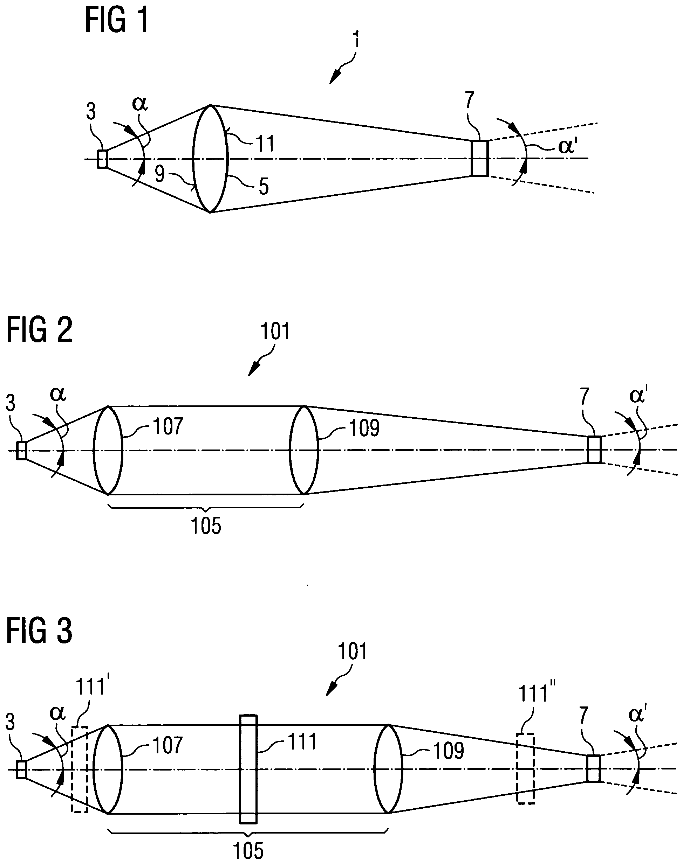

Ein erstes Ausführungsbeispiel für eine erfindungsgemäße Lichtquellenanordnung wird nachfolgend mit Bezug auf Figur 1 beschrieben. Die Figur zeigt den einfachsten Aufbau der erfindungsgemäßen Lichtquellenanordnung 1, der lediglich eine Leuchtdiode 3 als primäre Lichtquelle und eine Sammellinse 5 umfasst. Die Sammellinse 5 stellt die Abbildungsoptik der Lichtquellenanordnung dar, mit deren Hilfe ein reelles Bild 7 der Leuchtdiode 3 erzeugt wird. Die optischen Parameter der Sammellinse 5, d.h. ihr Brechungsindex und die Krümmungen der Linsenflächen sind so gewählt, dass der Vergrößerungsmaßstab des Bildes 7 im Vergleich zur Leuchtdiode 3 als dem Urbild zwischen 1,5 und 2,5 liegt und insbesondere zwischen 2 und 2,5. Dies führt dazu, dass die Leuchtfläche des Bildes 7 1,5 mal bis 2,5 mal so groß ist wie die Leuchtfläche der Leuchtdiode 3 und insbesondere 2 mal bis 2,5 mal so groß. Im vorliegenden Ausführungsbeispiel beträgt der Vergrößerungsmaßstab 2, d.h. der Öffnungswinkel α des von der Leuchtdiode 3 emittierten Strahlenbündels ist doppelt so groß wie der Öffnungswinkel α' des von dem Bild 7 ausgehenden Strahlenbündels. Außerdem ist die Leuchtfläche des Bildes 7 doppelt so groß wie die Leuchtfläche der Leuchtdiode 3.

Wenn man davon ausgeht, dass eine Leuchtdiode mit einem Öffnungswinkel von rund ± 60° abstrahlt, beträgt der Öffnungswinkel α', mit dem das Bild 7 der Leuchtdiode abstrahlt ± 30°, was in etwa dem Abstrahlwinkel des Austrittsendes einer Lichtleitfaser entspricht. Bei einer Lichtquellenanordnung mit einem Vergrößerungsmaßstab von ca. 2 kann daher das Bild 7 der Leuchtdiode 3 anstelle des Austrittsendes einer Lichtleitfaser in einer Beleuchtungsvorrichtung für ein medizinisch-optisches Beobachtungsgerät angeordnet werden.

Ein zweites Ausführungsbeispiel für die erfindungsgemäße Lichtquellenanordnung ist in Figur 2 dargestellt. Elemente, die sich nicht vom ersten Ausführungsbeispiel unterscheiden, sind mit denselben Bezugsziffern, wie in Figur 1 bezeichnet und werden nicht noch einmal erläutert.

Die Lichtquellenanordnung gemäß dem in Figur 2 dargestellten zweiten Ausführungsbeispiel unterscheidet sich von der in Figur 1 dargestellten Lichtquellenordnung dadurch, dass die Abbildungsoptik 105 in Form eines Doppelkollektors mit zwei Sammellinsen 107, 109 ausgebildet ist. Die Abbildungsoptik 105 erzeugt ein Bild 7 der Leuchtdiode 3 mit einem Abbildungsmaßstab von ca. zwei. Aber auch andere Abbildungsmaßstäbe, insbesondere Abbildungsmaßstäbe zwischen 1,5 und 2,5 und insbesondere zwischen 2 und 2,5 sind möglich.

Die in Figur 2 nur schematisch dargestellten Linsen des Doppelkollektors 105 können doppelkonvexe und/oder plankonvexe Linsen mit sphärischen und/oder asphärischen Linsenflächen sein. Die leuchtdiodenseitig gelegene Linse 107 des Doppelkollektors ist so ausgestaltet, dass sie die Leuchtdiode 3 nach unendlich abbildet, also ein paralleles Strahlenbündel erzeugt. Dieses wird wiederum von der bildseitig gelegenen Linse 109 fokussiert, um das Bild 7 der Leuchtdiode 3 zu erzeugen.

Da zwischen den beiden Linsen 107, 109 des Doppelkollektors ein paralleles Strahlenbündel vorliegt, kann der Abstand zwischen den beiden Linsen 107, 109 variiert werden und es können zudem afokale optische Elemente, beispielsweise Spiegel oder Prismen zum Ablenken des Strahlenbündels, dazwischen angeordnet sein. Auf diese Weise wird eine große Freiheit zwischen der Anordnung der Leuchtdiode 3 als Lumineszenzstrahler und dem Bild 7 der Leuchtdiode 3 erreicht.

Im ersten Ausführungsbeispiel wie im zweiten Ausführungsbeispiel kommen weiße Leuchtdioden als breitbandige Lumineszenzstrahler zur Anwendung. Es besteht jedoch auch die Möglichkeit, statt breitbandiger Lumineszenzstrahler schmalbandige Lumineszenzstrahler, beispielsweise blaue oder rote Leuchtdioden, zu verwenden. Je nach medizinisch-optischem Gerät, für die die Lichtquellenanordnung Verwendung finden soll, können auch Lumineszenzstrahler Verwendung finden, die im nichtsichtbaren Spektralbereich emittieren, beispielsweise im ultravioletten oder infraroten Spektralbereich emittierende Leuchtdioden. Dies kann etwa sinnvoll sein, wenn im Beobachtungsobjekt mittels der Lichtquellenanordnung Fluoreszenz angeregt werden soll.

Aber auch, wenn das Beobachtungsobjekt mit weißem Beleuchtungslicht beleuchtet werden soll, kann ein schmalbandiger Lumineszenzstrahler, beispielsweise eine blaue Leuchtdiode oder eine im ultravioletten Spektralbereich emittierende Leuchtdiode, zur Anwendung kommen. Um dennoch breitbandiges Beleuchtungslicht, insbesondere weißes Beleuchtungslicht, zur Verfügung stellen zu können, ist die Lichtquellenanordnung dann mit einem Konverterelement ausgestattet, welches wenigstens einen Teil des von der Leuchtdiode emittierten Lichtes in Licht mit einer längeren Wellenlänge umwandelt.

Eine Lichtquellenanordnung mit einem Konverterelement ist in Figur 3 dargestellt. Der grundsätzliche Aufbau der in Figur 3 dargestellten Lichtquellenanordnung entspricht dem der in Figur 2 dargestellten Lichtquellenanordnung. Es ist lediglich ein Konverterelement 111 zwischen den beiden Sammellinsen 107, 109 des Doppelkollektors 105 angeordnet. Grundsätzlich kann das Konverterelement 111 aber auch zwischen der Leuchtdiode 3 und der leuchtdiodenseitigen Linse 107 oder zwischen der bildseitigen Linse 109 und dem Bild 7 angeordnet sein, wie in Figur 3 durch die Bezugsziffern 111' und 111" angedeutet ist. Insbesondere im letztgenannten Fall können Farbfehler auch ohne achromatische oder apochromatische Linsen vermieden werden, da die Abbildungsoptik 105 nur von einer schmalen Wellenlängenverteilung durchsetzt wird.

Das Konverterelement 111 ist mit einem Konverterleuchtstoff ausgestattet, der wenigstens einen Anteil des schmalbandigen Lichtes der Leuchtdiode 3 in Licht mit einer längeren Wellenlänge umwandelt. Wenn beispielsweise die Leuchtdiode 3 blaues Licht emittiert, kann der Konverterleuchtstoff so gewählt sein, dass er einen Teil des blauen Lichtes in gelbes Licht umwandelt, so dass die Überlagerung des gelben Lichtes mit dem verbleibenden blauen Licht weißes Licht ergibt. Wenn dagegen beispielsweise eine UV-Strahlung emittierende Leuchtdiode Verwendung findet, ist es möglich, die UV-Strahlung mittels des Konverterleuchtstoffes vollständig in Licht im sichtbaren Spektralbereich umzuwandeln. Zudem ist es möglich, durch Verwendung mehrerer, hintereinander in der Lichtquellenanordnung eingebrachter oder einbringbarer Konverterelemente mit verschiedenen Konverterleuchtstoffen oder mittels eines Konverterelements mit einem Konverterleuchtstoffgemisch die UV-Strahlung vollständig in Licht mit wenigstens zwei, in der Summe zur breitbandigem oder weißem Licht führenden Wellenlängenverteilungen umzuwandeln. Das Verwenden von hintereinander in der Lichtquellenanordnung angeordneter oder in die Lichtquellenanordnung einbringbarer Konverterelemente oder eines in der Lichtquellenanordnung angeordneten oder in die Lichtquellenanordnung einbringbaren Konverterelementes mit einem Leuchtstoffgemisch ist aber nicht nur bei Verwendung einer in UV-Bereich emittierenden LED, sondern auch bei Verwendung einer im sichtbaren Spektralbereich emittierenden LED grundsätzlich möglich.

Insbesondere, wenn das Konverterelement oder die Konverterelemente der Lichtquellenanordnung austauschbar ausgestaltet ist bzw. sind, kann die spektrale Wellenlängenverteilung des vom Bild 7 emittierten Lichtes in weiten Bereichen eingestellt werden, beispielsweise um weißes Licht mit unterschiedlichen Farbtemperaturen realisieren können und/oder Licht mit weißen und nicht weißen Wellenverteilungen.

Um die Effizienz des Konverterelementes z erhöhen, kann dieses an seiner der Leuchtdiode zugewandten Fläche mit einer dichroitischen Schicht versehen sein, die für das Licht der Leuchtdiode durchlässig, für konvertiertes Licht jedoch hoch effektiv ist.

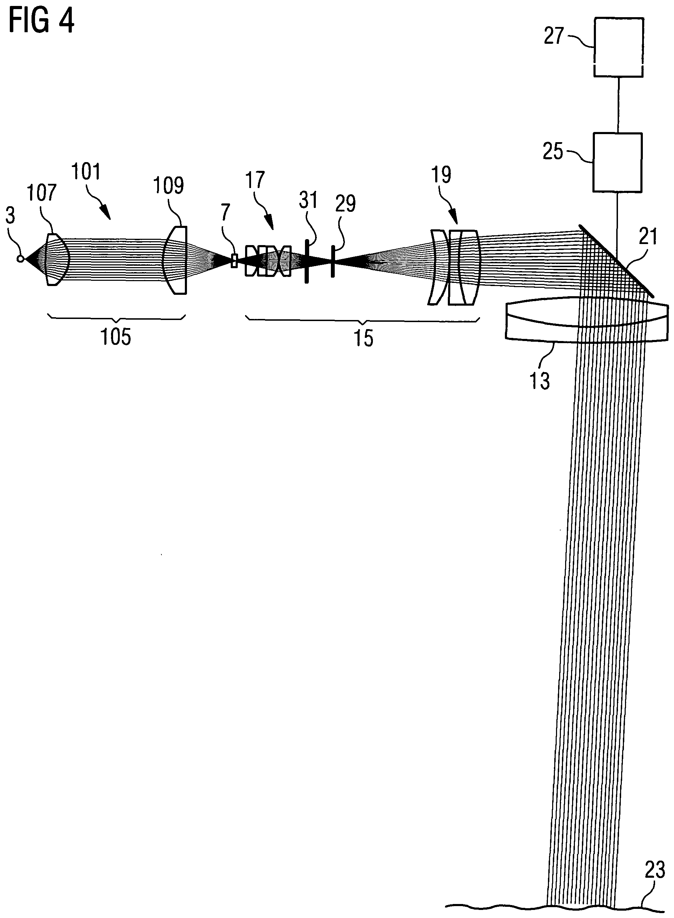

Ein Operationsmikroskop als Beispiel für ein medizinisch-optisches Beobachtungsgerät mit einer Beleuchtungsvorrichtung, die eine erfindungsgemäße Lichtquellenanordnung umfasst, ist in Figur 4 in einer schematischen Seitenansicht dargestellt. Neben einer erfindungsgemäßen Lichtquellenanordnung 1, die im Aufbau im Wesentlichen der in Figur 2 beschriebenen Lichtquellenanordnung entspricht, zeigt die Figur das Hauptobjektiv 13 des Operationsmikroskops sowie eine Beleuchtungsoptik 15, die eine Kollektoroptik 17 und eine Kondensoroptik 19 umfasst. Im vorliegenden Ausführungsbeispiel sind sowohl die Kollektoroptik 17 als auch die Kondensoroptik 19 aus Linsengruppen aufgebaut, um Abbildungsfehler im Beleuchtungsstrahlengang soweit wie möglich zu reduzieren. Über einen Strahlteiler, beispielsweise einen teildurchlässigen Spiegel 21, wird der Beleuchtungsstrahlengang in das Hauptobjektiv 13 eingekoppelt und über das Hauptobjektiv dem Beobachtungsobjekt 23 zugeführt.

Die Lichtquellenvorrichtung 1 umfasst in Figur 4 asphärische Linsenflächen, um Abbildungsfehler im Bild 7 der Leuchtdiode 3 möglichst gering zu halten.

Neben dem Beleuchtungsstrahlengang, welcher die optischen Elemente Kollektor 17, Kondensor 19, Strahlteiler 21 und Hauptobjektiv 13 umfasst, weist das Operationsmikroskop einen Beobachtungsstrahlengang auf. Dieser verläuft vom Beobachtungsobjekt 23 ausgehend durch das Hauptobjektiv 13 und den Strahlteiler 21, wobei der Beobachtungsstrahlengang im Unterschied zum Beleuchtungsstrahlengang vom Strahlteiler 21 nicht abgelenkt wird. Im Beobachtungsstrahlengang schließt sich an den Strahlteiler 21 ein Vergrößerungseinstellelement 25 an, mit dem sich der Vergrößerungsfaktor, mit dem im Beobachtungsstrahlengang eine Vergrößerung erfolgt, einstellen lässt. Das Vergrößerungseinstellelement 25 kann insbesondere als Zoomsystem ausgebildet sein, in dem mindestens drei Linsen oder Linsengruppen vorhanden sind, wobei zwei Linsen bzw. Linsengruppen entlang der optischen Achse verschiebbar sind, so dass sich der Vergrößerungsfaktor stufenlos einstellen lässt. Alternativ ist es auch möglich, das Vergrößerungseinstellelement 25 als stufigen Vergrößerungswechsler auszugestalten. In einem solchen sind mehrere Linsengruppen vorhanden, wobei die Linsen einer Linsengruppe in einer fest vorgegebenen Anordnung zueinander stehen. Ein Wechsel des Vergrößerungsfaktors erfolgt in einem solchen stufigen Vergrößerungswechsler durch wechselweises Einbringen verschiedener derartiger Linsengruppen in den Beobachtungsstrahlengang.

Die Vergrößerungseinstelleinrichtung 25 kann bereits als zweikanalige Optik ausgebildet sein, d.h. sie weißt einen linken und einen rechten stereoskopischen Teilstrahlengang auf, wobei jeder Teilstrahlengang eigene optische Elemente aufweist. Alternativ kann die Vergrößerungseinstelleinrichtung aber auch als sogenannte "große Optik" ausgebildet sein, d.h. ihre optischen Elemente sind so groß, dass sie gleichzeitig von beiden stereoskopischen Teilstrahlengängen durchsetzt werden.

An die Vergrößerungseinstelleinrichtung 25 schließt sich dann ein rein optischer oder ein optisch/eiektronischer Binokuiartubus 27 an. in einem rein optischen Binokulartubus 27 sind in jedem stereoskopischen Teilstrahlengang ein Tubusobjektiv und ein Okular angeordnet. Mittels der Tubusobjektive werden in den stereoskopischen Teilstrahlengängen jeweils Zwischenbilder generiert, die mittels der Okularoptik nach unendlich abgebildet werden, so dass ein Betrachter die Zwischenbilder mit entspanntem Auge beobachten kann. In einem kombinierten optischen und elektronischen Binokulartubus 27 befindet sich in jedem stereoskopischen Teilstrahlengang eine Abbildungsoptik, welche das Beobachtungsobjekt 23 auf zwei elektronische Bildsensoren abbildet.

Im vorliegenden Ausführungsbeispiel ist die Beleuchtungsvorrichtung des Operationsmikroskops als sogenannte Köhlersche Beleuchtung ausgebildet. In einer solchen wird die Lichtquelle, also das Bild 7 der Leuchtdiode 3, in eine Zwischenbildebene abgebildet, in der sich in der Regel eine Aperturblende 29 befindet, mit deren Hilfe sich die Helligkeit der Beleuchtung gezielt einstellen lässt. Weiterhin ist eine Leuchtfeldblende 31 vorhanden, die sich im Beobachtungsstrahlengang in einer zur Objektebene des Beobachtungsobjekts 23 konjugierten Ebene befindet. Objekte, die in einer solchen konjugierten Ebene angeordnet sind, werden in der Objektebene scharf abgebildet. Mittels der Leuchtfeldblende 31 kann daher eine scharfe Begrenzung des Leuchtfeldes im Objekt realisiert werden. Insgesamt lässt sich mit einer Köhlerschen Optik ein scharf begrenztes homogenes Leuchtfeld im Objekt 23 generieren. Die in Figur 4 dargestellte Beleuchtungsoptik entspricht im Wesentlichen der in DE 10 2006 013 761 A1 beschriebenen Beleuchtungsoptik mit dem Unterschied, dass statt dem dort beschriebenen Lichtleitfaseraustrittsende als Lichtquelle das Bild 7 der Leuchtdiode 3 dient.

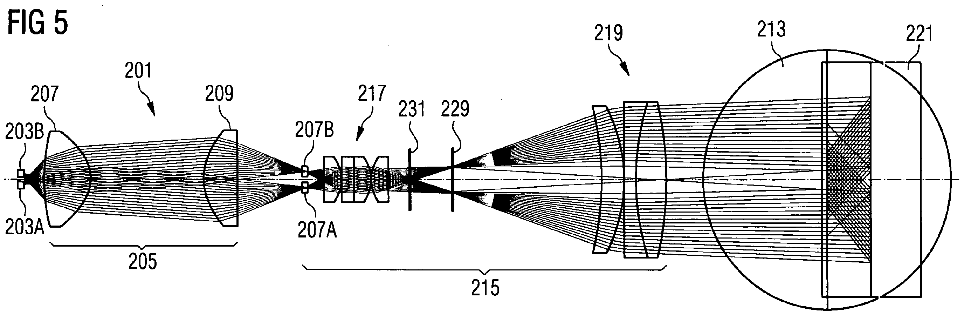

Figur 5 zeigt eine Abwandlung des in Figur 4 dargestellten Operationsmikroskops in einer Draufsicht. Das in Figur 5 dargestellte Ausführungsbeispiel unterscheidet sich sowohl in der Lichtquellenanordnung 201 als auch in der Beleuchtungsoptik 215 von dem in Figur 1 dargestellten Ausführungsbeispiel dadurch, dass es zum Realisieren einer Koaxialbeleuchtung ausgebildet ist. In einer solchen Koaxialbeleuchtung umfasst die Beleuchtung zwei Teilbeleuchtungsstrahlengänge, die mittels eines Strahlteilers 221 koaxial zu den stereoskopischen Teilbeobachtungsstrahlengängen dem Objekt zugeführt werden.

In dem in Figur 5 dargestellten Ausführungsbeispiel umfasst die Lichtquellenanordnung 201 zum Erzeugen der Koaxialbeleuchtung zwei Lumineszenzstrahler, nämlich zwei Leuchtdioden 203A, 203B, sowie einen Doppelkollektor 205 mit zwei Sammellinsen 207, 209, welche als große Linsen ausgebildet sind, d.h. die Linsen werden sowohl von dem von der Leuchtdiode 203A als auch von dem von der Leuchtdiode 203B ausgehenden Licht durchsetzt, um Bilder 207A, 207B der Leuchtdioden zu erzeugen. Die Linsenflächen sind asphärisch, um das Erzeugen der beiden Bilder mit möglichst geringen Abbildungsfehlern zu ermöglichen. Der Abstand zwischen den beiden Bildern 207A, 207B wird wie die Leuchtfläche und der Abstrahlwinkel mit dem Vergrößerungsmaßstab der Abbildungsoptik vergrößert, so dass durch geeignetes Einstellen des Abstandes zwischen den beiden Leuchtdioden 203A, 203B ein geeigneter Abstand zwischen den beiden Bildern 207A, 207B realisiert werden kann.

Die Beleuchtungsoptik 215 ist ebenfalls als große Optik ausgebildet, d.h. sowohl für den vom Bild 207A ausgehenden Strahlengang als auch für den vom Bild 207B ausgehenden Strahlengang ist jeweils ist eine gemeinsame Kollektoroptik 217 und eine gemeinsame Kondensoroptik 219 vorhanden. Lediglich die in der Zwischenbildebene der Beleuchtungsoptik 215 befindliche Aperturblende 229 und die in der zur Objektebene konjugierten Ebene befindliche Leuchtfeldblende 231 sind als Doppelblenden ausgestattet, d.h. sie weisen jeweils eine Blendenöffnung für jeden Teilstrahlengang der Beleuchtung auf.

Mittels des Strahlteilers 221 werden die beiden Teilstrahlengänge der Beobachtungsoptik dann durch das Hauptobjektiv 213 hindurch in Richtung auf das Beobachtungsobjekt abgelenkt. Eine derartige Koaxialbeleuchtung kommt beispielsweise bei ophthalmologischen Operationsmikroskopen häufig zum Erzeugen einer Rotreflexbeleuchtung zur Anwendung.

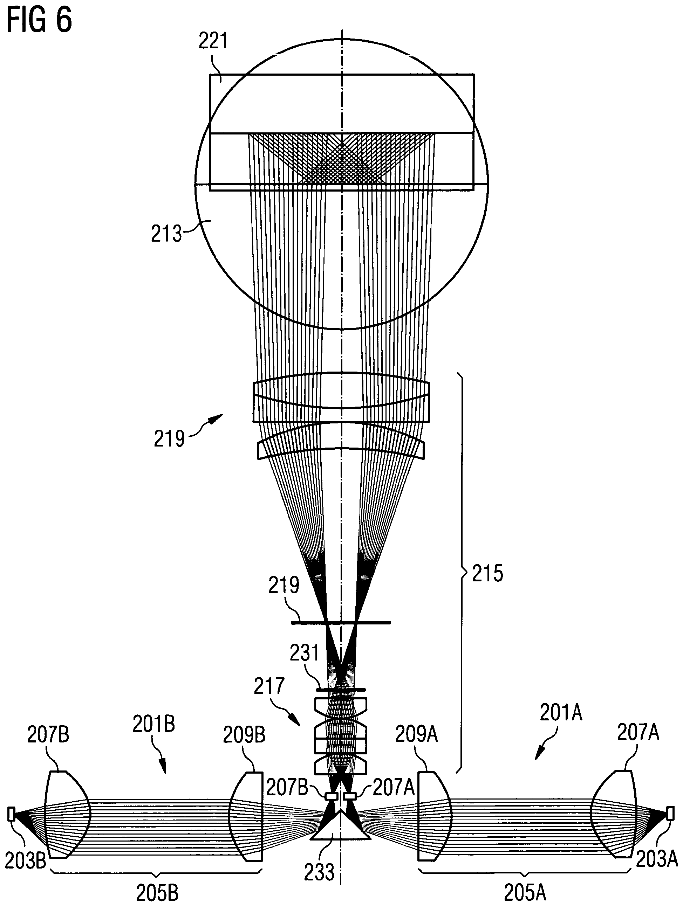

Eine alternative Realisierung der Koaxialbeleuchtung ist in Figur 6 in einer Draufsicht dargestellt. Die Beleuchtungsvorrichtung 215 sowie der Strahlteiler 221 und das Hauptobjektiv 213 unterscheiden sich nicht von den entsprechenden Elementen aus Figur 5 und sind daher mit denselben Bezugsziffern wie in Figur 5 bezeichnet.

Die in Figur 6 gezeigte Abwandlung unterscheidet sich von dem in Figur 5 dargestellten Operationsmikroskop dadurch, dass statt einer Lichtquellenanordnung 201 mit zwei Lumineszenzstrahlern 203A, 203B zwei Lichtquellenanordnungen 201A, 201B zur Anwendung kommen, welche jeweils nur einen einzigen Lumineszenzstrahler 203A, 203B in Form von Leuchtdioden umfassen. Jedem Lumineszenzstrahler 203A, 203B ist eine eigene Abbildungsoptik 205A, 205B zugeordnet, die jeweils der in Figur 4 gezeigten Abbildungsoptik 105 entspricht.

Die beiden Lichtquellenanordnungen 201A, 201B sind im dargestellten Ausführungsbeispiel einander gegenüberliegend in einem Winkel von 90° zur optischen Achse der Beleuchtungsoptik 215 angeordnet, d.h. die optischen Achsen der Doppelkollektoren 205A, 205B der beiden Lichtquellenanordnungen 201A, 201B weisen einen Winkel von 90° zur optischen Achse der Beleuchtungsoptik 215 auf. Mittels eines Dreieckspiegels 233 werden die beiden Strahlenbündel der Lichtquellenanordnungen um 90° abgelenkt, damit die Bilder 207A, 207B der Leuchtdioden 203A, 203B in die Beleuchtungsoptik 215 eingekoppelt werden können.

Obwohl die optischen Achsen der Lichtquellenanordnungen 201A, 201B in Figur 6 einen Winkel von 90° zur optischen Achse der Beleuchtungsoptik 215 aufweisen, können die Lichtquellenanordnungen 201A, 201B auch mit einem anderen Winkel relativ zur optischen Achse der Beleuchtungsoptik 215 angeordnet sein. Der Winkel des Dreieckspiegels 233 ist dann entsprechend anzupassen.

Während die in Figur 5 dargestellte Variante mit einer einzigen, zwei Leuchtdioden umfassenden Lichtquellenanordnung den Vorteil bietet, dass lediglich ein einziger Doppelkollektor vorhanden sein muss, bietet die in Figur 6 dargestellte Variante die Möglichkeit, Standardleuchtdioden in jeder Lichtquellenanordnung 201A, 201B zu verwenden. In der in Figur 5 dargestellten Variante ist dagegen unter Umständen eine Spezialanfertigung der beiden LEDs 203A, 203B erforderlich, um ihre Leuchtflächen nahe genug aneinander zu bringen, damit der Abstand zwischen den Bildern 207A, 207B für die gewünschte Anwendung nicht zu groß wird.

Die Erfindung wurde zu Erläuterungszwecken an konkreten Ausführungsbeispielen beschrieben. Von diesen Ausführungsbeispielen kann jedoch abgewichen werden. So können zwischen den beiden Linsen des Doppelkollektors beispielsweise Spiegel oder Prismen vorhanden sein, um den Strahlengang zu falten. Dadurch kann die Lichtquellenanordnung in ihrer Baulänge verkürzt werden.

Die erfindungsgemäße Lichtquellenanordnung ermöglicht das Verwenden von Leuchtdioden als primären Lichtquellen. Durch geeignete Wahl der Abbildungsoptik können Bilder der Lumineszenzstrahler mit einem Vergrößerungsmaßstab im Bereich zwischen 1,5x und 2,5x erzeugt werden, die dann als Beleuchtungslichtquelle der Beleuchtungsoptik in einem medizinisch-optischen Beobachtungsgerät zum Einsatz kommen könne. Gegenüber den Ausgangsenden von Lichtleitfasern als Beleuchtungslichtquellen bietet die erfindungsgemäße Lichtquellenanordnung eine homogenere Lichtausbreitung.

## Patentansprüche

1. Lichtquellenanordnung (1, 101, 201) zur Verwendung in einer eine Beleuchtungslichtquelle (7, 207) und eine Beleuchtungsoptik (15, 215) zur Beleuchtung eines Beobachtungsobjekts (23) mit Beleuchtungslicht der Beleuchtungslichtquelle (7, 207) aufweisenden Beleuchtungsvorrichtung eines medizinisch-optischen Beobachtungsgeräts, wobei die Lichtquellenanordnung (1, 101, 201) wenigstens einen Lumineszenzstrahler (3, 203) als primäre Lichtquelle umfasst, wobei der Lumineszenzstrahler (3, 203) eine Leuchtdiode ist, wobei die Lichtquellenanordnung (1, 101, 201) eine Abbildungsoptik (5, 105, 205) umfasst, die ein Bild (7, 207) des wenigstens einen Lumineszenzstrahlers (3, 203) mit einem definierten Vergrößerungsmaßstab erzeugt, welches die Beleuchtungslichtquelle für die Beleuchtungsvorrichtung bildet, **dadurch gekennzeichnet, dass** das Bild (7, 207) des wenigstens einen Lumineszenzstrahlers (3, 203) mit einem Vergrößerungsmaßstab im Bereich zwischen 1,5x und 2,5x gebildet wird.

2. Lichtquellenanordnung (1, 101, 201) nach Anspruch 1, **dadurch gekennzeichnet, dass** sie als Abbildungsoptik einen Doppelkollektor (105, 205) aufweist.

3. Lichtquellenanordnung (1, 101, 201) nach Anspruch 1 oder Anspruch 2, **dadurch gekennzeichnet, dass** die Abbildungsoptik (5, 105, 205) wenigstens eine asphärische Linse aufweist.

4. Lichtquellenanordnung (1, 101, 201) nach einem der Ansprüche 1 bis 3, **dadurch gekennzeichnet, dass** die Abbildungsoptik (5, 105, 205) eine achromatische oder apochromatische Linse umfasst.

5. Lichtquellenanordnung (1, 101, 201) nach einem der Ansprüche 1 bis 4, **dadurch gekennzeichnet, dass** sie einen breitbandiges Licht emittierenden Lumineszenzstrahler umfasst.

6. Lichtquellenanordnung nach einem der Ansprüche 1 bis 5, **dadurch gekennzeichnet, dass** sie einen schmalbandiges Licht emittierenden Lumineszenzstrahler (3, 203) umfasst.

7. Lichtquellenanordnung (101) nach Anspruch 6, **dadurch gekennzeichnet, dass** sie ein Konverterelement (111) umfasst, welches mit einem Konverterleuchtstoff zum Konvertieren wenigstens eines Teils des schmalbandigen Lichtes des Lumineszenzstrahlers (3) versehen ist und zwischen dem wenigstens einen Lumineszenzstrahler (3) und dem Bild (7) des wenigstens einen Lumineszenzstrahlers (3) in die Lichtquellenanordnung (101) eingebracht oder einbringbar ist.

8. Lichtquellenanordnung (101) nach Anspruch 7, **dadurch gekennzeichnet, dass** das Konverterelement (111) zwischen der Abbildungsoptik und dem Bild (7) des wenigstens einen Lumineszenzstrahlers (3) in die Lichtquellenanordnung (101) eingebracht oder einbringbar ist.

9. Lichtquellenanordnung nach Anspruch 7 oder Anspruch 8, **dadurch gekennzeichnet, dass** das wenigstens eine Konverterelement (111) eine dem Lumineszenzstrahler (3) zugewandte Eintrittsfläche für das von dem Lumineszenzstrahler (3) ausgehende Licht aufweist, die mit einer dichroitischen Schicht versehen ist, die für in das Konverterelement (111) eintretendes Licht mit der Wellenlängenverteilung des von dem Lumineszenzstrahler (3) emittierten Lichtes transparent ist und für in Richtung auf den Lumineszenzstrahler (3) gerichtetes konvertiertes Licht hoch reflektiv ist.

10. Beleuchtungsvorrichtung für ein für ein medizinisch-optisches Beobachtungsgerät, welche eine Beleuchtungslichtquelle (7, 207) und eine Beleuchtungsoptik (15, 215) zur Beleuchtung eines Beobachtungsobjekts (23) mit Beleuchtungslicht der Beleuchtungslichtquelle (7, 207) aufweist, **dadurch gekennzeichnet, dass** sie wenigstens eine Lichtquellenanordnung (101, 201) nach einem der Ansprüche 1 bis 9 aufweist, wobei das mit der Abbildungsoptik (5, 105, 205) der Lichtquellenanordnung (101, 201) erzeugte Bild (7, 207) des wenigstens einen Lumineszenzstrahlers (3, 203) die Beleuchtungslichtquelle für die Beleuchtungsvorrichtung bildet.

11. Beleuchtungsvorrichtung nach Anspruch 10, **dadurch gekennzeichnet, dass** sie wenigstens zwei Beleuchtungslichtquellen (7A, 7B) aufweist und beide Beleuchtungslichtquellen durch dieselbe Lichtquellenanordnung (201) gebildet sind.

12. Beleuchtungsvorrichtung nach Anspruch 10, **dadurch gekennzeichnet, dass** sie wenigstens zwei Beleuchtungslichtquellen (7A, 7B) aufweist und jede Beleuchtungslichtquelle (7A, 7B) durch eine eigene Lichtquellenanordnung (201A, 201B) gebildet ist.

13. Verwendung einer Lichtquellenanordnung (1, 101, 201) nach einem der Ansprüche 1 bis 9, wobei das mittels der Abbildungsoptik (5, 105, 205) gebildete Bild (7, 207) des Lumineszenzstrahlers (3, 203) die Beleuchtungslichtquelle für die Beleuchtungsoptik (15, 215) eines medizinisch-optischen Beobachtungsgeräts bildet und der Lumineszenzstrahler (3, 203) eine Leuchtdiode ist, **dadurch gekennzeichnet, dass** das Bild (7, 207) des wenigstens einen Lumineszenzstrahlers (3, 203) von der Abbildungsoptik (5, 105, 205) mit einem Vergrößerungsmaßstab im Bereich zwischen 1,5x und 2,5x gebildet wird.

## Claims

1. Light source arrangement (1, 101, 201) for use in an illumination apparatus of a medical-optical device, said illumination apparatus having an illumination light source (7, 207) and an illumination optical unit (15, 215) for illuminating an observation object (23) with illumination light from the illumination light source (7, 207), wherein the light source arrangement (1, 101, 201) comprises at least one luminescence emitter (3, 203) as a primary light source, wherein the luminescence emitter (3, 203) is a light emitting diode, wherein the light source arrangement (1, 101, 201) comprises an imaging optical unit (5, 105, 205), which generates an image (7, 207) of the at least one luminescence emitter (3, 203) with a defined magnification scale, said image forming the illumination light source for the illumination apparatus, **characterized in that** the image (7, 207) of the at least one luminescence emitter (3, 203) is formed with a magnification scale in the range of between 1.5x and 2.5x.

2. Light source arrangement (1, 101, 201) according to Claim 1, **characterized in that** it has a double collector (105, 205) as an imaging optical unit.

3. Light source arrangement (1, 101, 201) according to Claim 1 or Claim 2, **characterized in that** the imaging optical unit (5, 105, 205) has at least one aspherical lens element.

4. Light source arrangement (1, 101, 201) according to any one of Claims 1 to 3, **characterized in that** the imaging optical unit (5, 105, 205) comprises an achromatic or apochromatic lens element.

5. Light source arrangement (1, 101 201) according to any one of Claims 1 to 4, **characterized in that** it comprises a luminescence emitter which emits broadband light.

6. Light source arrangement according to any one of Claims 1 to 5, **characterized in that** it comprises a luminescence emitter (3, 203) which emits narrowband light.

7. Light source arrangement (101) according to Claim 6, **characterized in that** it comprises a converter element (111) which is provided with a converter phosphor for converting at least some of the narrowband light of the luminescence emitter (3) and which is introduced or introducible into the light source arrangement (101) between the at least one luminescence emitter (3) and the image (7) of the at least one luminescence emitter (3).

8. Light source arrangement (101) according to Claim 7, **characterized in that** the converter element (111) is introduced or introducible into the light source arrangement (101) between the imaging optical unit and the image (7) of the at least one luminescence emitter (3).

9. Light source arrangement according to Claim 7 or Claim 8, **characterized in that** the at least one converter element (111) has an entry face, facing the luminescence emitter (3), for the light emanating from the luminescence emitter (3), said interface being provided with a dichroic layer which is transparent to the light entering into the converter element (111) with the wavelength distribution of the light emitted by the luminescence emitter (3) and which is highly reflective for converted light directed in the direction of the luminescence emitter (3).

10. Illumination apparatus for a medical-optical observation device, which has an illumination light source (7, 207) and an illumination optical unit (15, 215) for illuminating an observation object (23) with illumination light from the illumination light source (7, 207), **characterized in that** it has at least one light source arrangement (101, 201) according to any one of Claims 1 to 9, wherein the image (7, 207) of the at least one luminescence emitter (3, 203), generated by the imaging optical unit (5, 105, 205) of the light source arrangement (101, 201), forms the illumination light source for the illumination apparatus.

11. Illumination apparatus according to Claim 10, **characterized in that** it has at least two illumination light sources (7A, 7B) and both illumination light sources are formed by the same light source arrangement (201).

12. Illumination apparatus according to Claim 10, **characterized in that** it has at least two illumination light sources (7A, 7B) and each illumination light source (7A, 7B) is formed by a dedicated light source arrangement (201A, 201B).

13. Use of a light source arrangement (1, 101, 201) according to any one of Claims 1 to 9, wherein the image (7, 207) of the luminescence emitter (3, 203), formed by means of the imaging optical unit (5, 105, 205), forms the illumination light source for the illumination optical unit (15, 215) of a medical-optical observation device and the luminescence emitter (3, 203) is a light-emitting diode, **characterized in that** the image (7, 207) of the at least one luminescence emitter (3, 203) is formed by the imaging optical unit (5, 105, 205) with a magnification scale in the range of between 1.5x and 2.5x.

## Revendications

1. Arrangement de sources de lumière (1, 101, 201) destiné à être utilisé dans un dispositif d'éclairage d'un appareil d'observation médico-optique, possédant une source de lumière d'éclairage (7, 207) et une optique d'éclairage (15, 215), en vue d'éclairer un objet observé (23) avec de la lumière d'éclairage de la source de lumière d'éclairage (7, 207), l'arrangement de sources de lumière (1, 101, 201) comportant au moins un projecteur à luminescence (3, 203) comme source de lumière primaire, le projecteur à luminescence (3, 203) étant une diode électroluminescente, l'arrangement de sources de lumière (1, 101, 201) comportant une optique de représentation (5, 105, 205) qui génère une image (7, 207) de l'au moins un projecteur à luminescence (3, 203) avec une échelle de grossissement définie, laquelle forme la source de lumière d'éclairage pour le dispositif d'éclairage, **caractérisé en ce que** l'image (7, 207) de l'au moins un projecteur à luminescence (3, 203) est formée avec une échelle de grossissement dans la plage entre 1,5x et 2,5x.

2. Arrangement de sources de lumière (1, 101, 201) selon la revendication 1, **caractérisé en ce qu'**il possède un double collecteur (105, 205) en tant qu'optique de représentation.

3. Arrangement de sources de lumière (1, 101, 201) selon la revendication 1 ou la revendication 2, **caractérisé en ce que** l'optique de représentation (5, 105, 205) possède au moins une lentille asphérique.

4. Arrangement de sources de lumière (1, 101, 201) selon l'une des revendications 1 à 3, **caractérisé en ce que** l'optique de représentation (5, 105, 205) comporte une lentille achromatique ou apochromatique.

5. Arrangement de sources de lumière (1, 101, 201) selon l'une des revendications 1 à 4, **caractérisé en ce qu'**il comporte un projecteur à luminescence émettant une lumière à large bande.

6. Arrangement de sources de lumière selon l'une des revendications 1 à 5, **caractérisé en ce qu'**il comporte un projecteur à luminescence (3, 203) émettant une lumière à bande étroite.

7. Arrangement de sources de lumière (101) selon la revendication 6, **caractérisé en ce qu'**il comporte un élément convertisseur (111) qui est pourvu d'une substance luminescente destinée à convertir au moins une partie de la lumière à bande étroite du projecteur à luminescence (3) et qui est incorporé ou peut être incorporé dans l'arrangement de sources de lumière (101) entre l'au moins un projecteur à luminescence (3) et l'image (7) de l'au moins un projecteur à luminescence (3) .

8. Arrangement de sources de lumière (101) selon la revendication 7, **caractérisé en ce que** l'élément convertisseur (111) est incorporé ou peut être incorporé dans l'arrangement de sources de lumière (101) entre l'optique de représentation et l'image (7) de l'au moins un projecteur à luminescence (3).

9. Arrangement de sources de lumière selon la revendication 7 ou la revendication 8, **caractérisé en ce que** l'au moins un élément convertisseur (111) possède une surface d'entrée faisant face au projecteur à luminescence (3) pour la lumière émanant du projecteur à luminescence (3), laquelle est pourvue d'une couche dichroïque qui est transparente pour la lumière pénétrant dans l'élément convertisseur (111) avec la distribution de longueurs d'onde de la lumière émise par le projecteur à luminescence (3) et fortement réfléchissante pour la lumière convertie dirigée dans la direction du projecteur à luminescence (3).

10. Dispositif d'éclairage pour un appareil d'observation médico-optique, possédant une source de lumière d'éclairage (7, 207) et une optique d'éclairage (15, 215) en vue d'éclairer un objet observé (23) avec de la lumière d'éclairage de la source de lumière d'éclairage (7, 207), **caractérisé en ce qu'**il possède au moins un arrangement de sources de lumière (101, 201) selon l'une des revendications 1 à 9, l'image (7, 207) de l'au moins un projecteur à luminescence (3, 203) formée avec l'optique de représentation (5, 105, 205) de l'au moins un arrangement de sources de lumière (101, 201) formant la source de lumière d'éclairage pour le dispositif d'éclairage.

11. Dispositif d'éclairage selon la revendication 10, **caractérisé en ce qu'**il possède au moins deux sources de lumière d'éclairage (7A, 7B) et les deux sources de lumière d'éclairage sont formées par le même arrangement de sources de lumière (201).

12. Dispositif d'éclairage selon la revendication 10, **caractérisé en ce qu'**il possède au moins deux sources de lumière d'éclairage (7A, 7B) et chaque source de lumière d'éclairage (7A, 7B) est formée par un arrangement de sources de lumière (201A, 201B) propre.

13. Utilisation d'un arrangement de sources de lumière (1, 101, 201) selon l'une des revendications 1 à 9, l'image (7, 207) du projecteur à luminescence (3, 203) formée au moyen de l'optique de représentation (5, 105, 205) formant la source de lumière d'éclairage pour l'optique d'éclairage (15, 215) d'un appareil d'observation médico-optique et le projecteur à luminescence (3, 203) étant une diode électroluminescente, **caractérisée en ce que** l'image (7, 207) de l'au moins un projecteur à luminescence (3, 203) est formée par l'optique de représentation (5, 105, 205) avec une échelle de grossissement dans la plage entre 1,5x et 2,5x.
